Europäisches Patentamt

European Patent Office

Office européen des brevets

(11) Publication number: **0 136 804 B1**

# (12) EUROPEAN PATENT SPECIFICATION

(45) Date of publication of patent specification: **09.10.91**   (51) Int. Cl.⁵: **C12P 7/18**

(21) Application number: **84305748.0**

(22) Date of filing: **22.08.84**

(54) **Industrial-scale process for the production of polyols by fermentation of sugars.**

(30) Priority: **24.08.83 GB 8322750**
**11.10.83 GB 8327192**

(43) Date of publication of application:
**10.04.85 Bulletin 85/15**

(45) Publication of the grant of the patent:
**09.10.91 Bulletin 91/41**

(84) Designated Contracting States:
**AT BE CH DE FR GB IT LI NL SE**

(56) References cited:

**APPLIED MICROBIOLOGY, vol. 12, no. 3, May 1964, pages 240-246, American Society for Microbiology, US; G.J. HAJNY et al.: "Erythritol production by a yeastlike fungus"**

**APPLIED MICROBIOLOGY, vol. 24, no. 5, pages 831-833, American Society for Microbiology, US; L. HANSSENS et al.: "pH-Dependent polyol production in Moniliella tomentosa"**

**APPLIED AND ENVIRONMENTAL MICROBIOLOGY, vol. 32, no. 1, July 1976, pages 56-63, American Society for Microbiology, US; L. HANSSENS et al.: "Types of respiratory activity in Moniliella tomentosa during growth under different conditions"**

(73) Proprietor: **CPC INTERNATIONAL INC.**
**International Plaza P.O. Box 8000**
**Englewood Cliffs New Jersey 07632(US)**

(72) Inventor: **de Troostenbergh, Jean-Claude**
**Cleerbeekmolen 305**
**B-3215 Houwaart(BE)**
Inventor: **Reuliaux, Luc**
**Langewej 69**
**B-1900 Overijse(BE)**
Inventor: **Oudenne, Françoise**
**Rue Louis Hab 200, Bte 8**
**B-1040 Brussels(BE)**

(74) Representative: **Wilkinson, Stephen John et al**
**Stevens, Hewlett & Perkins 1 St. Augustine's Place**
**Bristol BS1 4UD(GB)**

## Description

The invention relates to an industrial-scale process for the production of glycerol and/or erythritol and/or ribitol, by aerobic fermentation of a sugar with a sugar-tolerant fungus such as Moniliella tomentosa var. pollinis.

It is known that aerobic fermentation of a suitable sugar by the yeast-like fungus Moniliella tomentosa var. pollinis will produce erythritol; this was first reported by G.J. Hajny, J.H. Smith and J.C. Garver in Applied Microbiology 12, pp 240-246 (May 1964), who designated the microbe as Torula I₂A. Later, in Antonie van Leeuwenhoek 37, pp 107-118 (1971), L. Dooms, G.L. Hennebert and H. Verachtert classified the fungus as Moniliella tomentosa var. pollinis, and confirmed its ability to produce erythritol; the authors also set forth a complete morphological description of the microbe. In addition, they recognised that Moniliella can exist under two forms, yeast- and mould-like forms, according, essentially, to the applied culture conditions; on page 110 they state, "On agar slants both forms are produced. In stationary liquid media the mould-like form with abundant mycelium and more arthrospores than blastospores develops.

In shake-flask cultures, the yeast-like form predominates with rounded, oval and rectangular cells produced by budding and by fission while no mycelium is formed".

In translating these laboratory processes to an industrial scale, it is clearly necessary to maximise the yield of the desired product(s). One significant factor which emerged from our investigations of the process was the concentration of the sugar in the reaction medium and by manipulating this concentration as hereinafter described, we found that the final polyol concentration in the fermentation medium could be increased.

During our investigations of the process we did not observe any inhibition of the process by the polyols produced even in the range of polyol concentrations of 25% - 30% (w/v). Unfortunately, to achieve this high final product concentration, a starting sugar concentration of about 50% is necessary. When sugar concentrations higher than 35% were used, however, we found that growth of the microorganism was retarded, a fact which we attribute to the high osmotic pressure of the medium.

The present invention comprises a process which enables a high final product concentration to be achieved and, at the same time, avoids any inhibition of cell growth.

Accordingly, the present invention is an industrial process for the production of glycerol and/or erythritol and/or ribitol, by the aerobic fermentation of a suitable sugar by Moniliella tomentosa var. pollinis in which the total amount of the sugar added to the fermentation medium is in the range 40% to 80% weight/volume of the medium, the amount at the start of the fermentation being 20 to 35% and sugar being progressively added to the medium so that at the end of the fermentation, the total amount of sugar which has been added is at least 40% weight/volume.

Suitable sugars for use in the process are dextrose (glucose), sucrose, fructose and maltose, dextrose being particularly preferred.

In a development of the process, we have found that, if the sugar for the fermentation is provided in the form of a starch hydrolysate, the fermentation medium may have a relatively large dry substance without the growth of the Moniliella tomentosa being inhibited. We attribute this effect to larger molecular weight components of the hydrolysate which generate a relatively lower osmotic pressure. In this development, a maltodextrin is added to the fermentation medium at the start of the fermentation at a concentration greater than 40% dissolved solids, the maltodextrin being progressively converted in situ to a sugar fermentable by the Moniliella tomentosa var.pollinis by including glucoamylase in the fermentation medium. With this form of the invention, the sugar substrate is made available progressively in the fermentation medium by the activity of the glucoamylase enzyme.

The desired products of the process according to the present invention are erythritol and/or ribitol. Prior art workers have reported production of glycerol and arabitol, in addition to erythritol, from the fermentation of sugars by Moniliella tomentosa var. pollinis. In accordance with our process, no arabitol is produced, but ribitol, in addition to erythritol and glycerol is obtained, these three being the only polyols formed. Typically, the fermentation produces a product comprising ribitol, glycerol and erythritol in the following proportions, based on totaly polyols : ribitol 1%-20%; glycerol 5%-40%; the balance being erythritol.

Moniliella tomentosa var. pollinis is available at the Centraal Bureau voor Schimmelcultures (CBS), Baarn, The Netherlands, as CBS 461.67, and was also deposited and is available to the public at the Commonwealth Mycological Institute Culture Collection (CMI cc), Ferry Lane, Kew, Richmond, Surrey TW9 3AF, United Kingdom, under the number (CMI cc) 271648.

The microbial cells are cultivated on a solid medium containing malt extract (4%), yeast extract (0.2%) and agar (2%). The culture so formed is then inoculated to a sterilised medium containing sugar and a nitrogen source.

Throughout the specification and claims, all percentages and parts are by volume unless otherwise stated.

Air is supplied to the fermentor at a rate which depends on the size of the fermentation vessel and the degree of agitation of the contents of the vessel, but is generally in the range of 0.1 to 1.5 litre air/litre fermentation medium/minute. For example, in a 2-litre vessel, the air flow rate was 1 litre/litre/minute at a stirrer speed of 400 to 800 rpm, while in a 600-litre vessel, the rate was 0.5 to 1.0 litre/litre/minute without agitation other than that produced by the air flow. The fermentation is conducted at a temperature of between 27°C and 32°C, at a starting pH of between 3 and 6, preferably 4 to 5. The fermentation is stopped when the sugar has been consumed.

In the prior art process, as described by Hajny, various sources of nitrogen were described for use in the fermentation, for example, yeast extract, urea, corn steep liquor, malt sprouts, black strap molasses, malt extract and distillers dry solubles. In the process of the present invention good results are achieved with 0.5% yeast extract plus 0.1% urea or with 2% corn steep liquor plus 0.02% urea.

The starting pH should be between about 3.0 and 6.0 which decreases to about 2.0 to 3.5 during the fermentation. According to L. Hanssens, A. Van Regenmortel and H. Verachtert, Applied Microbiology, Vol. 24, No.5, pp 831-833 (November 1972), laboratory fermentations held at different constant pH's result in substantial differences in yields of total polyols and erythritol. We have found that, when working with larger scale fermentations (2-litre fermentors or larger) the total polyol and erythritol yields are less sensitive to starting pH within the 4.0 to 6.0 range. In order to avoid or minimise problems of contamination, a starting pH of 4 to 5 is preferred. The fermentation is conducted until all of the sugar has been consumed (fermentation times will normally be between 4 and 12 days, depending upon the amount of sugar used), after which the culture is stopped and the cells are removed from the culture broth, e.g. by centrifuging. The cell-free culture broth, which contains erythritol, ribitol and glycerol, can be used as such, with or without refining (e.g. by ultrafiltration and demineralisation), for certain applications, e.g. in the polymer industry. The refined culture broth can also be concentrated to 60% to 80% dissolved solids and the erythritol crystallised therefrom, e.g. by the technique described by J.M. Roxburg, J.F.T. Spencer and H.R. Sallens, Canadian Journal of Technology, Vol. 34, pp 248-353 (1956). The liquor remaining after recovery of the erythritol crystals, which is also a mixture of (non-crystallised)erythritol,ribitol and glycerol, can also be used after appropriate treatment.

The process according to the invention is advantageously carried out in the presence of the polysaccharide Xanthan gum which has the property of retaining the cells of the Moniliella tomentosa var. pollinis in the fermentation medium and reducing their loss via the foam which occurs during the fermentation. Preferably 100 to 500 ppm Xanthan gum may be present and excellent results are obtained with about 300 ppm. More gum may be used than is necessary for retaining the cells in the fermentation medium (i.e. more than about 500 ppm) but such excess is wasteful.

The overall process is also improved by including in the fermentation medium a conventional antifoam agent. Synthetic antifoam agents (e.g. silicone type or fatty alcohols) are preferred over the natural (e.g. lard oil) products because they can be used in smaller quantities and the final fermentation broth does not need extensive refining for their removal. With most synthetic commercial antifoam agents, 200-300 or 400 ppm is sufficient for optimum foam control.

The following examples are intended to illustrate the practice of the invention.

Preparation of the seed culture

500 ml erlenmeyers containing 50 ml culture medium comprising 20% dextrose, 0.5% yeast extract, 0.1% urea and 300 ppm Xanthan gum were inoculated with a colony from a solid medium and cultivation was conducted for 3 to 4 days at a starting pH of 5, a temperature of 30°C and under reciprocating agitation of 100 oscillations/minute. These cultures could be used as such to carry out a fermentation or could be used to inoculate larger cultures for propagation of the biomass for larger fermentations.

Example 1

A 600-litre fermentor containing 450 litres fermentation medium comprising 32% dextrose, 2% corn steep liquor, 0.02% urea, 300 ppm SAG 471 antifoam (a dimethylpolysiloxane from Union Carbide) and 300 ppm Xanthan gum was inoculated with 9% of a seed culture. The air supply was 0.7 litre air per litre culture medium per minute. The starting pH was 3.9. From the fourth day to the ninth day the fermentor was fed continuously with dextrose and urea until the total amount of dextrose added to the fermentor was 50% by weight of the initial volume of the fermentation medium and the total amount of urea added to the fermentor was 0.1%. After 13 days, the dextrose was fully consumed and the final erythritol concentration was 16% and the total polyol concentration was 26.4%.

Example 2

Three 50 ml erlenmeyer flasks each contained 50 ml fermentation medium comprising 0.5% yeast extract, 0.1% urea and, respectively, 30%, 40% and 50% maltodextrin of dextrose equivalent (D.E.) 12. Each flask also contained 0.1% weight/weight based on maltodextrin of glucoamylase. The flasks were inoculated with 9% of a seed culture and held at a temperature of 30°C under a reciprocating agitation of 100 oscillations/minute. The fermentations were conducted for 7, 10 and 14 days and, at the end of these times, the solutions were analysed. The results are given in the following Table.

|  |  | 30% malto-dextrin | 40% malto-dextrin | 50% malto-dextrin |
|---|---|---|---|---|
| Time of fermentation |  | 7 days | 10 days | 14 days |
| Ribitol | g/litre | 2.5 | 3.1 | 6.75 |
| Erythritol | g/litre | 96.5 | 127.2 | 149.7 |
| Glycerol | g/litre | 3.7 | 4.1 | 12.7 |
| Ethanol | g/litre | 3.6 | 4.3 | 3.4 |
| Total polyols | g/litre | 102.7 | 134.4 | 169.15 |

**Claims**

1.  An industrial process for the production of glycerol and/or erythritol and/or ribitol by the aerobic fermentation of a suitable sugar by Moniliella tomentosa var. pollinis characterised in that the total amount of the sugar added to the fermentation medium is in the range 40% to 80% weight/volume of the medium, the amount of sugar added at the start of the fermentation being 20 to 35% weight/volume and sugar being progressively added to the medium so that, at the end of the fermentation, the total amount of sugar which has been added is at least 40% weight/volume.

2.  A process according to claim 1 characterised in that the sugar is dextrose, sucrose, fructose, or maltose.

3.  A process according to claim 1 characterised in that the sugar is provided in the form of a starch hydrolysate and a glucoamylase enzyme which progressively converts the hydrolysate to the sugar.

4.  A process according to any one of the preceding claims characterised in that air or an oxygen/inert gas composition approximating to air is fed to the fermentation at a flow rate between 0.1 and 1.5 litre/litre fermentation medium/minute.

5.  The process of any one of the preceding claims characterised in that the pH at the beginning of the fermentation is adjusted to between 3 and 6, preferably between 4 and 5.

6.  The process of any one of the preceding claims characterised in that Xanthan gum is present in an amount between 100 ppm and 500 ppm of the fermentation medium.

7.  The process of any one of the preceding claims characterised in that a conventional antifoam agent is also added to the fermentation.

4

**Revendications**

1. Procédé industriel de production de glycérol et/ou d'érythritol et/ou de ribitol par fermentation aérobie d'un sucre convenable par Moniliella tomentosa var. pollinis, caractérisé en ce que la quantité totale de sucre ajoutée au milieu de fermentation se situe dans la plage de 40 à 80 % en poids/volume du milieu, la quantité de sucre ajoutée au début de la fermentation étant de 20 à 35 % en poids/volume et du sucre étant progressivement ajouté au milieu de manière qu'à la fin de la fermentation, la quantité totale de sucre qui a été ajoutée soit d'au moins 40 % en poids/volume.

2. Procédé suivant la revendication 1, caractérisé en ce que le sucre est le dextrose, le saccharose, le fructose ou le maltose.

3. Procédé suivant la revendication 1, caractérisé en ce que le sucre est introduit sous la forme d'un hydrolysat d'amidon et d'une glucoamylase qui convertit progressivement l'hydrolysat en sucre.

4. Procédé suivant l'une quelconque des revendications précédentes, caractérisé en ce que de l'air ou un mélange d'oxygène et de gaz inerte dont la composition se rapproche de celle de l'air est chargé dans le milieu de fermentation à une vitesse comprise entre 0,1 et 1,5 litre/litre de milieu de fermentation/minute.

5. Procédé suivant l'une quelconque des revendications précédentes, caractérisé en ce que le pH au début de la fermentation est ajusté entre 3 et 6, de préférence entre 4 et 5.

6. Procédé suivant l'une quelconque de revendications précédentes, caractérisé en ce que de la gomme xanthane est présente en une quantité comprise entre 100 ppm et 500 ppm du milieu de fermentation.

7. Procédé suivant l'une quelconque des revendications précédentes, caractérisé en ce qu'un agent antimoussant classique est aussi ajouté au milieu de fermentation.

**Patentansprüche**

1. Verfahren zur Herstellung von Glycerin und/oder Erythrit und/oder Ribit in industriellem Umfang durch aerobe Gärung eines geeigneten Zuckers mit Moniliella tomentosa var. pollinis, dadurch gekennzeichnet, daß die Gesamtmenge des dem Gärmedium zugesetzten Zuckers im Bereich von 40% bis 80% (Gew./Vol.) des Mediums liegt, wobei die Menge am Beginn der Gärung 20 bis 35% (Gew./Vol.) beträgt und Zucker allmählich zum Medium zugesetzt wird, so daß am Ende der Gärung die gesamte Zuckermenge, die zugesetzt wurde, mindestens 40% (Gew./Vol.) beträgt.

2. Verfahren nach Anspruch 1, dadurch gekennzeichnet, daß der Zucker Dextrose, Sucrose, Fructose oder Maltose ist.

3. Verfahren nach Anspruch 1, dadurch gekennzeichnet, daß der Zucker in Form eines Stärkehydrolysats und eines Glucoamylase-Enzyms eingesetzt wird, der das Hydrolysat allmählich in Zucker umwandelt.

4. Verfahren nach einem der vorhergehenden Ansprüche, dadurch gekennzeichnet, daß Luft oder eine annähernd der Luft entsprechende Sauerstoff-Inertgas-Zusammensetzung in die Gärung in einer Menge von 0.1 bis 1.5 Liter/Liter Gärmedium/Minute zugeführt wird.

5. Verfahren nach einem der vorhergehenden Ansprüche, dadurch gekennzeichnet, daß der pH am Anfang der Gärung auf 3 bis 6, vorzugsweise 4 bis 5, eingestellt wird.

6. Verfahren nach einem der vorhergehenden Ansprüche, dadurch gekennzeichnet, daß Xanthangummi in einer Menge von 100 ppm bis 500 ppm im Gärmedium vorhanden ist.

7. Verfahren nach einen der vorhergehenden Ansprüche, dadurch gekennzeichnet, daß auch ein konventionelles Antischaummittel zur Gärung zugesetzt wird.